(19) Europäisches Patentamt
European Patent Office
Office européen des brevets

(11) Publication number: **0 322 206 B1**

(12) # EUROPEAN PATENT SPECIFICATION

(45) Date of publication of patent specification: **24.05.95**

(51) Int. Cl.6: **A61K 31/52**, A61K 31/70, A61K 38/02

(21) Application number: **88312107.1**

(22) Date of filing: **21.12.88**

The file contains technical information submitted after the application was filed and not included in this specification

(54) **Compositions and methods for improving cold tolerance in animals and humans.**

(30) Priority: **21.12.87 GB 8729714**

(43) Date of publication of application: **28.06.89 Bulletin 89/26**

(45) Publication of the grant of the patent: **24.05.95 Bulletin 95/21**

(84) Designated Contracting States: **AT CH DE FR GB LI SE**

(56) References cited:

**AMERICAN JOURNAL OF PHYSIOLOGY, vol. 21, no. 4, April 1987, pages 737-742,American Physiological Society; T.F. LEE et al.: "Enhancement of cold-stimulated thermogenesis in the corpulent rat (LA/N cp) by aminphylline"**

**JOURNAL OF APPLIED PHYSIOLOGY, vol. 63, no. 2, August 1987, pages 589-596,American Physiological Society; L.C.H. WANG et al.: "Metabolic and hormonalresponses in theophylline-increased cold resistance in males"**

(73) Proprietor: **HER MAJESTY IN RIGHT OF CANADA AS REPRESENTED BY THE MINISTER OF NATIONAL DEFENCE**

**Ottawa**
**Ontario K1A OK2 (CA)**

(72) Inventor: **Wang, Lawrence Chia-Huang**
**5012-144 Street**
**Edmonton**
**Alberta T6H 4H3 (CA)**
Inventor: **Lee, Tze-Fun**
**4218-149 Street**
**Edmonton**
**Alberta T6H 5L9 (CA)**

(74) Representative: **Crampton, Keith John Allen et al**
**D. YOUNG & CO.**
**21 New Fetter Lane**
**London EC4A 1DA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

PHARMACOLOGY BIOCHEMISTRY AND BEHAVIOR, vol. 25, no. 4, October 1986, pages769-773; J.M. CARNEY et al.: "Differential antagonism of behavioral depressantand hypothermic effects of 5'-(N-ethylcarboxamide) adenosine by theobromine"

RESEARCH COMMUNICATIONS IN CHEMICAL PATHOLOGY AND PHARMACOLOGY, vol. 16, no. 4,April 1977, pages 621-628, Westbury, US; C.M. OUIRCE et al.: "Drug interactionson body temperature maintenance in the mouse"

MINERVA MEDICA, vol. 76, 1985, pages 1401-1405, IT; C. MACCHIONE et al.:"Haemorheological, haemocoagulative and photoplethysmographic changes inducedby the administration of pentoxyphyllin in subjects with Raynaud's disease"

INTERNATIONAL JOURNAL OF OBESITY, vol. 9, suppl. 2, 1985, pages 31-42, Lonon,GB; L.J. BUKOWIECKI: "Regulation of energy expenditure in brown adipose tissue"

IRCS MEDICAL SCIENCE, vol. 9, no. 5, May 1981, page 431; E. PORSCHE et al.:"Forced swimming in rats: Temperature regulation, immobility and the effects of1- (5'-oxohexyl)-3-methyl-7-propyl-xanthine-HWA 285"

PSYCHOPHARMACOLOGY, vol. 58, no. 2, 1978, pages 161-166, Springer-Verlag,

Berlin, DE; C.-J. ESTLER et al.: "Swimming capacity of mice after prolongedtreatment with psychostimulants"

L.J. Bukowiecki et al., Am. J. Physiol., 1983, Vol. 244, R500-R507, "Effects of sucrose, caffeine, and cola beverages on obesity, cold resistence, and adipose tissue cellularity"

## Description

This invention relates to improving cold tolerance and prevention of accidental hypothermia in animals and humans.

When exposed to severe cold, mammals increase their heat production to counter heat loss in order to maintain a constant body temperature. If heat loss surpasses maximum heat production, hypothermia results and unless aided by exogenous heat, death ensues. Given that defence operations and emergencies can involve prolonged cold exposure, any treatment that may enhance thermogenesis would be beneficial in reducing or eliminating the danger of becoming hypothermic.

Theophylline (THEO) and aminophylline (AMPY; 85% theophylline, 15% ethylenediamine) have been shown to elevate maximum thermogenesis and to improve cold tolerance in animals and man in severe cold: J. App. Physiol. Vol 63(2), 1987, 589-596 and Am. J. Physiol. , Vol 21(4), 1987, 737-742. However, their mechanism of action is widely disputed. One school of thought is that competitive inhibition of phosphodiesterase (PDE) activity and thus increased levels of intracellular cAMP for enhanced substrate conversion and mobilization is responsible. Another involves competitive antagonism against adenosine receptors, thereby releasing the antilipolytic effect of adenosine after sympathetic stimulation in the cold. Since both of these known compounds are non-selective adenosine antagonists, i.e. they exhibit other pharmacological activity, the operating mechanism was uncertain.

In considering the possible role of adenosine in thermogenesis, adenosine is formed as an end product of ATP hydrolysis following physiological stimulation, e.g. adrenergic or local hypoxia. It is released into extracellular space and binds to adenosine receptors on the cell surface and exerts an inhibiting influence on substrate (fuel) mobilization. Since substrate availability is a critical limiting factor for full expression of heat producing capability in the cell, a shortage of fuel supply would lend less than full expression of thermogenic capability and reduced cold resistance. The use of adenosine antagonists should rectify this deficiency and amplify the body's own cold fighting ability.

To test the mechanism of adenosine antagonism, a known selective adenosine receptor antagonist, 8-phenyltheophylline (8-PT) which preferentially binds to adenosine receptors was used. To test the mechanism of PDE inhibition, a selective PDE inhibitor, enprofylline (ENPRO) which is devoid of adenosine antagonism was employed.

In Pharm. Biochem. Behav., Vol 25(4), 1986, 769-773 the thermogenic effect of theobromine is disclosed and is shown to be related to its activity as an adenosine receptor antagonist. However, those studies were carried out at 22°C and were aimed at ways of countering drug-induced hypothermia, that is to say to reverse the hypothermic effect and the locomotor activity-suppressing effect of the known adenosine receptor agonists $N^6$-cyclohexyl-adenosine (CHA) and 5'-(N-ethylcarboxamide) adenosine (NECA). Those studies showed that it was, indeed possible to blockade CHA-and NECA-induced hypothermia using theobromine as an adenosine receptor antagonist. However those studies did not show or indicate that theobromine had any thermogenic activity per se, whether by virtue of its activity as adenosine receptor antagonist, or otherwise, such that it might be used to combat environmentally induced, as opposed to drug-induced hypothermia. Thus based on Pharm. Biochem. Behav., cited above, there is nothing that would lead the person skilled in the art towards using theobromine, or any other adenosine receptor antagonist, for that matter, as a means of either treating environmentally induced hypothermia or improving the tolerance of the human or animal body to the cold.

Similarly with Int. J. Obes., Vol. 9, 1985, 31-42 which reviews the endocrinological and biochemical mechanisms controlling energy expenditure in brown adipose tissue at the cellular level and at total tissue level. Brown adipose tissue is the primary site for cold induced thermogenesis in mammals, and it is known that, whilst brown adipose tissue levels in man and other mammals living in temperate climates are low, brown adipose tissue levels in mammals living in cold climates are much higher, and that as man, and other animals, become acclimatised to colder temperatures, levels of brown adipose tissue do increase leading to increased thermogenesis, and hence acclimatisation of the body to the colder environment. The document shows that brown adipose tissue thermogenesis is stimulated by caffeine, theophylline and 3-isobutyl-1-methylxanthine all as a result of phosphodiesterase inhibition, not as a result of their adenosine receptor antagonist activity. Moreover, the in vitro studies reported in the document require extremely high levels (e.g. 5mM theophylline) of the phosphodiesterase inhibitor to bring about effective blocking of the enzyme and hence effective potentiation of brown adipose tissue thermogenesis. Bearing in mind that plasma theophylline levels in excess of about 0.2mM are usually fatal, the document again has little to teach the person skilled in the art with regard to an effective medication to prevent or minimize the effects of hypothermia in man or other animals due to physical exposure.

In Psychopharm., Vol 58, 1978, 161-166 caffeine is described as having a negative rather than positive effect on thermogenesis in mice and showed that treatment with caffeine results in a diminished cold tolerance in mice. But that was on the basis of chronic treatment, rather than an acute single dose treatment, that chronic treatment involving 150$\mu$g/g p.o. caffeine per day every day for six-weeks, and since those findings were on the basis of chronic treatment with high levels of caffeine, they are not necessarily inconsistent with other art, nor for that matter, the present invention based on the thermogenic effect of caffeine at acute, rather than chronic levels of administration.

Finally in the prior art, Am. J. Physiol., Vol. 233, 1983, R500-R507 describes the effects of sucrose, caffeine, and cold beverages on obesity, cold resistance and adipose tissue cellularity. The results showed that the cold resistance of rats was improved quite remarkably in Coca-Cola™ consumption that increased cold resistance being accompanied by a significant increase in brown adipose tissue weight, cellularity, triglyceride content, protein content and cytochrome oxidase activity. But since those effects were apparently not obtained by caffeine consumption, pR500 left hand column line 17 and pR504 left hand column lines 14-17, thermogenesis by caffeine, and hence the utility of caffeine and other adenosine receptor antagonists in the treatment of physiological hypothermia and in increasing the cold tolerance levels in man and other animals remains extremely equivocal.

In the studies reported above (J. App. Physiol. Vol.63(2), 1987, 589-596), theophylline was administered to rats in combination with a commercial nutrient mixture (ENSURE®) containing 14.8% protein, 31.5% fat and 53.7% carbohydrate.

In accordance with the present invention, it has been found that, in the oral administration of an adenosine antagonist other than theophylline or aminophylline alone in combination with a nutrient supplement as a means for improving cold tolerance and enhancing thermogenesis, the composition of the nutrient supplement is critically important.

More specifically best results are obtained using the nutrient supplement, a composition containing, oil a weight basis:

50 - 60% of a carbohydrate mixture consisting essentially of starch (St), sucrose (Su) and glucose (Gl), preferably in a St:Su:Gl weight ratio of 6.4:3:1 or 10.6:5:1,

15 - 50% protein, and

0 - 25% fat.

Representative adenosine receptor antagonists for use in accordance with this invention include xanthines of the structural formula

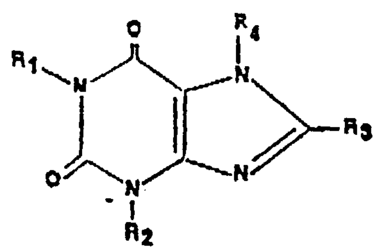

wherein $R_1$ and $R_2$ are H or $C_1$-$C_6$ alkyl group,
$R_4$ is H or $CH_3$, and $R_3$ is

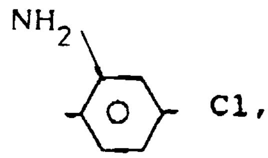

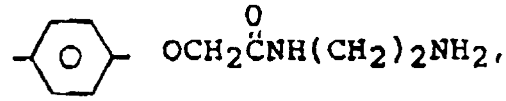

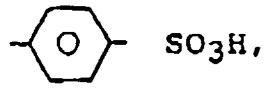

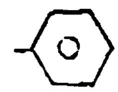 ,

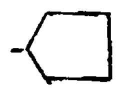 ,

H; provided that when $R_1$ and $R_2$ are both $CH_3$, $R_3$ and $R_4$ are not both H, and that when $R_1$, $R_3$ and $R_4$ are all H, $R_2$ is not propyl.

The $C_1$-$C_6$ alkyl groups $R_1$ and $R_2$ may each preferably contain from 1 to 5, and particularly 1 to 4, especially 1 to 3, carbon atoms.

Other suitable adenosine antagonists are: triazoloquinazoline, pyrazoloquinoline, pyrazolopyridine and imidazopyrimidine.

Amongst the xanthine compounds which are known to have adenosine antagonistic effects, i.e. the compounds of the above formula, the following have been tested for their increased thermogenic capability in man and animals and hence their utility in accordance with the present invention to provide improved cold tolerance in animals and humans:

8-cyclopentyltheophylline (CPT);
8-phenyltheophylline (8-PT);
8-(4-(2-aminoethyl)amino) carboxylmethyloxyphenyl)- 1,3-dipropylxanthine (XAC);
8-(2-amino-4-chlorophenyl)-1,3-dipropylxanthine (PACPX);
8-(p-sulfophenyl)-1,3-dipropyl xanthine (DPSPX);
caffeine (1,3,7-trimethylxanthine); and
theobromine (3,7-dimethylxanthine).

CPT has the advantage of being highly soluble in water and is thus the easiest to administer by injection.

Caffeine is also easily soluble in water but its adenosine antagonistic effect is not as strong as that of CPT. Theobromine is not very soluble in water but this can be circumvented by using both injection and oral adminstration. Because of their insolubility in water other test compounds were dissolved in a suitable organic solvent, e.g. polyethylene glycol (8-PT) or DMSO (XAC, PACPX and DPSPX). In these controls, injections were carried out using the solvent only.

The results are presented in the accompanying drawings in which:

Figure 1 is a graph which illustrates the thermogenic effect of injected 8-phenyltheophylline (8-PT) in mammals;
Figure 2 illustrates the thermogenic effect of injected 8-cyclopentyltheophylline (CPT);
Figure 3 illustrates the thermogenic effect of injected caffeine;
Figure 4 illustrates the thermogenic effect of injected theobromine;
Figure 5 illustrates the thermogenic effect of orally administered theobromine (in the form of cocoa);
Figure 6 illustrates the thermogenic effect of injected 8-(4-(2-aminoethyl)amino)-carboxylmethyloxyphenyl)-1,3-dipropylxanthine (XAC);
Figure 7 illustrates the thermogenic effect of injected 8-(2-amino-4-chlorophenyl)-1,3-dipropyl xanthine (PACPX);
Figure 8 illustrates the thermogenic effect of injected 8-(p-sulfophenyl)-1,3-dipropyl xanthine (DPSPX);
Figure 9 illustrates the thermogenic effect of enprofylline (ENPRO);
Figure 10 illustrates the thermogenic effect of 8-PT and AMPY when administered together;

Figure 12 illustrates the effect on cold tolerance of a female subject of theobromine (4 mg/kg p.o.) and a nutritional supplement in comparison with the nutritional supplement alone and a placebo and in accordance with the experimental protocol set out in Table 4;

Figure 13 illustrates the effect on cold tolerance of a male subject of theobromine (4 mg/kg p.o.) and a nutritional supplement using the same protocol;

Figure 14 illustrates the improved cold tolerance achieved in humans (3 males, 2 females), again using theobromine (4 mg/kg p.o.) and a nutritional supplement according to the protocol of Table 4;

Figure 15 illustrates the effect of theobromine (2 mg/kg p.o.) and a nutritional supplement on the cold tolerance of two male subjects against a placebo and in accordance with the protocol set out in Table 5; and

Figure 16 illustrates the effect of theobromine (in the form of cocoa powder containing 1.5% theobromine for an equivalent 2 mg/kg p.o.) plus a nutritional supplement on the cold tolerance of two subjects (one male, one female) against a placebo and in accordance with the experimental protocol set out in Table 6.

As can be seen from the results presented in the drawings CPT (Figure 2), PACPX (Figure 7) and DPSPX (Figure 8) significantly increased the heat producing capability of the rats and significantly increased their cold resistance as seen by the very much higher final body temperature after 2 hours of severe cold exposure. 8-PT also significantly increased thermogenesis and cold resistance in a dose-dependent manner (Figure 1). Because of the solubility problem, which presumably affects the absorption of the injected drug following its administration, the dosage difference between the CPT and 8-PT is about 5,000 fold reflecting their concentrational differences in in vitro receptor binding studies. Similarly, injection (i.p.) of caffeine at 20 mg/kg (Figure 3) and theobromine at 20 mg/kg (Figure 4) also demonstrated significant improvement on thermogenesis and cold resistance in rats. Interestingly, when theobromine is administered orally to rats (Figure 5), significant increase in thermogenesis was also observed. Oral theobromine was administered as cocoa powder and water. This is because cocoa powder contains about 1-2% of theobromine based on dry weight with little or no other xanthine compounds present as quantified by our HPLC analysis and as indicated in the open literature (e.g. Shively and Tarka, Jr., 1984, In: The methylxanthine beverages and foods: chemistry, consumption and health effects, edited by G.A. Spiller, New York: Allan Liss, p. 149-178). This indicates that through the oral route, even relatively water insoluble adenosine antagonists, such as theobromine may be quite useful as an agent for the stimulation thermogenesis and improvement of cold resistance. This has been verified in our initial studies in humans (Figures 12 to 16).

The result on XAC (Figure 6) are not as clean-cut as those for the other compounds; the main reason being the solubility problem. However, XAC showed a 7% increase in maximum and total thermogenesis, very close to the 10% or more found in the other compounds which elicited statistically significant increases.

Since some of the xanthine (e.g. caffeine, theobromine) used also possess antiphosphodiesterase enzyme (PDE) activity, it is a possibility that the action of these xanthines may be through PDE inhibition rather than adenosine antagonism. To dispel this possibility, we have selected enprofylline (3-propylxanthine), which is known to have PDE inhibitory effect ($IC_{50}$) = 100 - 400$\mu$M) but devoid of adenosine antagonism (Persson & Kjellin, Enprofylline, a principally new antiasmatic xanthine, Acta Pharmacol. Toxicol. 49:313-316, 1981) to test its influence on thermogenesis and cold resistance. The results indicated that enprofylline is without effect on stimulation of thermogenesis (Figure 9), confirming our contention that it is the antagonism of adenosine receptors but not the inhibition of PDE activity that is responsible for the beneficial effects of the many xanthines employed herein.

Our results also indicate that the maximum thermogenesis effect by optimal dose of 8-PT (5 mg/kg, i.p.) was significantly lower than that with the optimal dose of AMPY (18.7 mg/kg, i.p.) (Figure 10). Applicant has found that the deficit could be eradicated by combining optimal 8-PT dose with a lose dose of AMPY (1.25 mg/kg, i.p.). In fact, the thermogenic effect of 8-PT, when administered simultaneously with a low dose of AMPY (Figure 10), is markedly enhanced, and is increased to the same level as that after a high dose of AMPY. As the magnitude of HP induced by the combined drug treatment may have already reached the maximum aerobic capacity of the animal, it is difficult to distinguish whether these two compounds elicit thermogenesis in a synergistic or additive manner.

To demonstrate that a combination of adenosine antagonist and nutritional supplement is a better treatment than the use of adenosine antagonist or nutritional supplement alone, we have conducted experiments in rats verifying this prediction. Tables 1 and 2 below show that orally administered nutritional supplement alone, either in the form of different carbohydrate mixtures or as a mixture of carbohydrates, fat and protein, enhanced heat production and the magnitude and the duration of cold tolerance.

TABLE 1

| EFFECTS OF CARBOHYDRATE FEEDING ON COLD TOLERANCE IN RATS# | | | | |
|---|---|---|---|---|
| **Treatment (n-8)** | **Total HP (Kcal)** | **Max. HP (Kcal/15 min)** | **Change in Tb (°C)** | **Duration (min)** |
| Water 5 ml | 10.90±-0.91 | 1.50±0.03 | -10.83±0.76 | 144.4±6.98 |
| Sucrose 52% aq. solution | 14.31±1.27* (31%) | 1.66±0.04* (10%) | -8.68±0.69 | 163.1±7.23* 1 |
| Starch 55.2% aq. solution | 14.60±0.98* (34%) | 1.59±0.05 (6%) | -9.05±1.11 | 166.9±5.59* |
| Gl:Su:St (1:3:6.4) | 15.20±0.98* (39%) | 1.64±0.05* (9%) | -7.88±0.64* | 172.5±4.59* |
| Gl:Su:St (1:6:3.2) | 14.41±0.76* (32%) | 1.58±0.03 (5%) | -10.98±0.89 | 170.6±5.89* |
| Gl:Su:St (1:5:10.6) | 16.63±0.96* (53%) | 1.67±0.07* (11%) | -5.50±0.96* | 177.5±2.20* |
| Gl:Su:St (1:10:5.3) | 15.09±1.78* (39%) | 1.68±0.06* (11%) | -10.33±0.97 | 170.0±5.38* |

# Feeding was by gastric tube in a volume of 5 ml 15 min prior to cold exposure.
* Significantly different from water control, $p<0.05$ (Wilcoxin's Rank Test).
Number in brackets indicate percentage increase in heat production over control value.
Gl = Glucose; Su = Sucrose; St = Starch
The composition of carbohydrate is by weight ratio, wherein 1 = 0.26g.
The carbohydrate mixtures are dissolved in water.

TABLE 2

| EFFECTS OF SUBSTRATE FEEDING ON COLD TOLERANCE IN RATS# | | | | |
|---|---|---|---|---|
| **Treatment (n-)** | **Total HP (Kcal)** | **Max. HP (Kcal/15 min)** | **Change in Tb (°C)** | **Duration (min)** |
| Water 5 ml | 16.12±0.94 | 1.67±0.05 | -12.12±0.75 | 177.2±8.36 |
| Gl:Su:St (1:5:10.6) | 24.61±1.28* (53%) | 1.90±0.06* (14%) | -10.42±1.13 | 231.7±4.78* |
| Mixture (16 Kcal) | 25.83±1.64* (60%) | 2.01±0.07* (20%) | -8.52±1.83* | 230.0±4.08* |

# Feeding was by gastric tube in a volume of 5 ml either 15 min (for Gl:Su:St) or 30 min (for mixture) prior to cold exposure.
* Significantly different from water control, $p<0.05$ (Wilcoxin's Rank Test).
The composition of carbohydrate is by weight ratio.
Numbers in brackets indicate percentage increase in heat production over control value.
Gl = Glucose; Su = Sucrose; St = Starch
Composition of mixture = Gl:Su:St:Egg Albumin:Corn Oil - 1:3:6.4:5:0.33 (weight ratio, wherein 1 = 0.26g).
The mixtures are dissolved in water.
Composition of mixture in terms of weight percentages, carbohydrate = 66.2%, fat = 1.9% and protein = 31.9%.

However, as illustrated in Table 3 below, the combination of a general adenosine antagonist, theobromine (administered orally in the form of cocoa powder plus water) and carbohydrate mixtures resulted in the greatest improvement of heat production and cold tolerance as compared to the treatment using water, cocoa, or nutritional supplement alone. Consequently, the decrease of body temperature (Tb) was the least and the duration of cold exposure was the longest in adenosine antagonist and nutritional supplement treatment. It is therefore likely that this combination is the best for improving cold resistance and based on Tables 2 and 3, theobromine plus a nutritional mixture including carbohydrates, protein and fat is a desirable formula.

TABLE 3

| EFFECTS OF COCOA AND CARBOHYDRATE FEEDING ON COLD TOLERANCE IN RATS# | | | | |
|---|---|---|---|---|
| **Treatment (n-8)** | **Total HP (Kcal)** | **Max. HP (Kcal/15 min)** | **Change in Tb (°C)** | **Duration (min)** |
| Water 5 ml | 10.75±0.59 | 1.42±0.04 | -10.45±0.62 | 140.6±5.26 |
| Cocoa 1 g | 11.42±0.72 (6%) | 1.59±0.03* (12%) | -9.84±0.46 | 144.4±5.26 |
| Gl:Su:St (1:3:6.4) | 16.04±0.68* (49%) | 1.61±0.04* (13%) | -6.85±1.26* | 176.3±3.51* |
| Cocoa 1 g + Gl:Su:St (1:3:6.4) | 17.43±0.64* (61%) | 1.72±0.04* (21%) | -5.53±0.87* | 180.0±0.00* |
| Gl:Su:St (1:5:10.6) | 16.49±0.67* (32%) | 1.67±0.03 (5%) | -5.75±1.12* | 176.3±3.51* |
| Cocoa 1 g + Gl:Su:St (1:5:10.6) | 17.71±0.86* (65%) | 1.77±0.10* (25%) | -5.03±1.16* | 180.0±0.00* |

# Feeding was by gastric tube in a volume of 5 ml 15 min prior to cold exposure. The composition is by weight ratio, wherein 1 = 0.26g.
* Significantly different from water control, p<0.05 (Wilcoxin's Rank Test).
+ Significantly different from cocoa control, p<0.05 (Wilcoxin's Rank Test).
Numbers in brackets indicate percentage increase in heat production over water control group.
Gl = Glucose; Su = Sucrose; St = Starch
The mixtures are dissolved in water.

As seen in Tables 4 to 6 and Figures 12 to 16, initial experiments in humans of both sexes have shown that the combination of orally administered theobromine in amounts of 2 to 4 mg/kg of body weight and various combinations of nutritional supplements effective in improving cold tolerance and retarding the onset of hypothermia in severe cold.

With specific reference to Figure 12, the hatched boxes indicate duration of walking on a motorized treadmill at 6.4 km/h (4 mile/hr). Arrow indicates time of ingestion of either placebo drink or nutritional supplement. Theobromine is taken orally at time 0.

In Figure 13, the hatched boxes indicate duration of walking on a motorized treadmill at 8 km/hr (5 mile/hr). Arrow indicates time of ingestion of placebo drink and nutritional supplement. Arrows with cocoa on top indicate time of ingestion of cocoa plus nutritional supplement (total amount of theobromine in cocoa and nutritional supplement are equivalent to those in theobromine + nutritional supplement).

In Figure 14, the hatched boxes indicate duration of walking on a motorized treadmill at 6.4 km/hr (4 miles/hr) for females or 8km/hr (5 miles/hr) for males. Arrow indicates time of ingesting placebo drink or nutritional supplement.

Change in rectal temperature (°C) in 3 males and 2 females walking at 6.4 to 8 km/hr (4 to 5 miles/hr) intermittently (50% of time) at - 10°C for 3 hours dressed in shorts (males) or T-shirt and shorts (females) are reported in Table 4.

TABLE 4

CHANGE IN RECTAL TEMPERATURE

Treatment - Placebo and Placebo

| Time | M.B. | J.D. | S.B. | T.B. | B.M. | Average +/- se |
|---|---|---|---|---|---|---|
| | Subjects | | | | | |
| 0 | 0.00 | 0.00 | 0.00 | 0.00 | 0.00 | 0.000 +/- 0.000 |
| 30 | 0.10 | -0.10 | -0.40 | -0.20 | 0.00 | -0.040 +/- 0.092 |
| 60 | 0.00 | -0.80 | -0.70 | -0.10 | -0.40 | -0.400 +/- 0.141 |
| 90 | -0.20 | -1.10 | -0.70 | -0.40 | -1.10 | -0.700 +/- 0.162 |
| 120 | -0.60 | -1.10 | -1.00 | -0.60 | -1.30 | -0.920 +/- 0.125 |
| 150 | -0.90 | -1.10 | -1.30 | -0.70 | -1.60 | -1.120 +/- 0.140 |
| 180 | -1.10 | -1.15 | -1.20 | -0.90 | -1.70 | -1.210 +/- 0.119 |

Treatment - Pure Theobromine (4 mg/kg) and Nutritional Supplement (Nu. Sp.)

| Time | M.B. | J.D. | S.B. | T.B. | B.M. | Average +/- se |
|---|---|---|---|---|---|---|
| | Subjects | | | | | |
| 0 | 0.00 | 0.00 | 0.00 | 0.00 | 0.00 | 0.000 +/- 0.000 |
| 30 | 0.10 | -0.10 | -0.20 | 0.00 | 0.10 | -0.020 +/- 0.052 |
| 60 | -0.15 | -0.70 | -0.30 | -0.20 | -0.20 | -0.310 +/- 0.090 |
| 90 | -0.40 | -1.10 | -0.60 | -0.40 | -0.40 | -0.580 +/- 0.121 |
| 120 | -0.50 | -1.10 | -0.90 | -0.50 | -0.60 | -0.720 +/- 0.107 |
| 150 | -0.60 | -1.20 | -0.90 | -0.60 | -0.85 | -0.830 +/- 0.100 |
| 180 | -0.70 | -1.20 | -0.80 | -0.70 | -0.95 | -0.870 +/- 0.084 |

Nu.Sp.

| %/w | 355 KCal (1480 KJ) |
|---|---|
| 16.2 | 13.0 g Protein (casein and soya protein isolates) |
| 24.5 | 19.6 g Fat (corn oil and triglycerides) |
| 59.2 | 47.3 g Carbohydrate (corn syrup solids and sucrose) |

in 235 ml aq. solution.

Changes in rectal temperature (°C) in two males walking at 8 km/hr (5 miles/hr) intermittently (50% of time) at -10°C for 3 hours dressed in shorts are presented in Table 5.

TABLE 5

CHANGE IN RECTAL TEMPERATURE

Treatment - Placebo and Placebo

| Time | Subjects J.D. | Subjects S.B. |
|---|---|---|
| 0 | 0.00 | 0.00 |
| 30 | 0.05 | -0.40 |
| 60 | -0.45 | -0.70 |
| 90 | -0.85 | -0.70 |
| 120 | -1.00 | -1.00 |
| 150 | -1.05 | -1.10 |
| 180 | -1.10 | -1.20 |

Treatment - 2 mg/kg Theobromine and Nu.Sp.

| Time | Subjects J.D. | Subjects S.B. |
|---|---|---|
| 0 | 0.00 | 0.00 |
| 30 | 0.00 | 0.10 |
| 60 | -0.40 | -0.20 |
| 90 | -0.85 | -0.55 |
| 120 | -0.90 | -0.70 |
| 150 | -0.90 | -0.70 |
| 180 | -0.90 | -0.70 |

Nu.Sp. = Nutritional Supplement (same as Table 4)

| %/w | 355 KCal (1480 KJ) |
|---|---|
| 16.2 | 13.0 g Protein |
| 24.5 | 19.6 g Fat |
| 59.2 | 47.3 g Carbohydrate |

in 235 ml aq. solution.

Change in rectal temperature (°C) in one male and one female walking at 6.4 to 8 km/hr (4-5 miles/hr) intermittently (30% of time) at -10°C for 3 hours in shorts (males) or T-shirt and shorts (females) are presented in Table 6.

**TABLE 6**

**CHANGE IN RECTAL TEMPERATURE**

**Treatment** - Placebo and Placebo

| Time | Subjects A.B. | Subjects M.B. |
|---|---|---|
| 0 | 0.00 | 0.00 |
| 30 | -0.40 | -0.15 |
| 60 | -0.95 | -0.50 |
| 90 | -1.25 | -0.80 |
| 120 | -1.50 | -1.05 |
| 150 | -1.65 | -1.20 |
| | ** | -1.40 |

** Individual removed from cold due to reaching maximum allowable decrease in body temperature.

**Treatment** - CHOP 2 mg/kg Theobromine via Cocoa.

| Time | Subjects A.B. | Subjects M.B. |
|---|---|---|
| 0 | 0.00 | 0.00 |
| 30 | -0.50 | 0.00 |
| 60 | -0.65 | -0.30 |
| 90 | -0.75 | -0.50 |
| 120 | -0.85 | -0.80 |
| 150 | -0.95 | -0.90 |
| 180 | -0.90 | -1.00 |

CHOP - Carbohydrate Protein Supplement

%/w 250 KCal (1042 KJ)

| | | |
|---|---|---|
| 46.2 | 30.0 | Casein (calcium caseinate) |
| 5.2 | 3.4 g | Glucose |
| 15.5 | 10.1 g | Sucrose |
| 33.1 | 21.5 g | Corn Starch |
| | 9.0 g | Cocoa (containing 1.5%/w Theobromine) |

in 235 ml aq. solution.

## Claims

1. An orally ingestible composition for use in improving the tolerance of man or animals to a cold environment, or for use in the treatment of hypothermia caused by exposure of the human or animal body to a cold environment, said composition comprising a thermogenically effective amount of an adenosine receptor antagonist other than theophylline or aminophylline alone in admixture with a nutrient composition containing protein, carbohydrate and optionally fat, characterised in that the carbohydrate component of the nutrient composition comprises a thermogenic mixture of starch (St.), sucrose (Su) and glucose (Gl).

2. An orally ingestible composition according to claim 1, characterised in that the carbohydrate component of the nutrient composition is a mixture consisting essentially of starch, sucrose and glucose in a weight ratio of 6.4:3:1 or 10.6:5:1.

3. An orally ingestible composition according to claim 1 or 2 in which the nutrient composition contains on a weight basis 50-60% carbohydrate, 15-50% protein and 0-25% fat.

4. An orally ingestible composition according to claim 1, 2 or 3 wherein the adenosine receptor antagonist is a compound of the formula

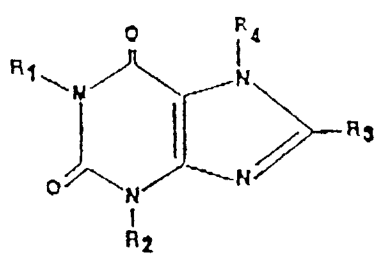

where each of $R_1$ and $R_2$ is a hydrogen atom or a $C_1$-$C_6$ alkyl group,
$R_4$ is a hydrogen atom or a methyl group,
and $R_3$ is

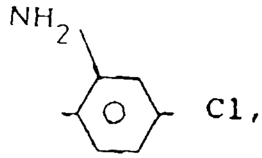

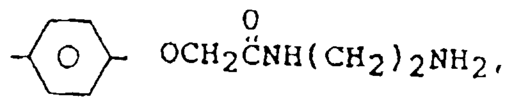

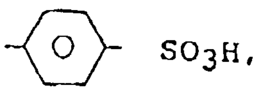

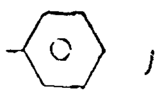

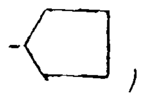

or H; provided that when $R_1$ and $R_2$ are both methyl, $R_3$ and $R_4$ are not both hydrogen, and that when $R_1$, $R_3$ and $R_4$ are all H, $R_2$ is not propyl.

5. An orally ingestible composition according to claim 1, 2 or 3 wherein the adenosine receptor antagonist is
   8-cyclopentyltheophylline (CPT);
   8-phenyltheophylline (8-PT);
   8-(4-(2-aminoethyl)amino)carboxylmethyloxyphenyl)-1,3-dipropylxanthine (XAC);
   8-(2-amino-4-chlorophenyl)-1,3-dipropylxanthine (PACPX);
   8-(p-sulfophenyl)-1,3-dipropyl xanthine (DPSPX);
   theobromine (3,7-dimethylxanthine).

6. An orally ingestible composition according to claim 1, 2 or 3 which comprises a mixture of starch, sucrose, glucose and cocoa.

**Patentansprüche**

1. Oral einnehmbare Zusammensetzung für die Verwendung bei der Verbesserung der Toleranz von Menschen und Tieren gegenüber kalter Umgebung oder für die Verwendung bei der Behandlung von Hypothermie, die dadurch verursacht ist, daß der Menschen- oder Tierkörper einer kalten Umgebung ausgesetzt wurde, wobei diese Zusammensetzung eine thermogenetisch wirksame Menge eines von Theophyllin oder Aminophyllin allein verschiedenen Adenosinrezeptorantagonisten im Gemisch mit Protein, Kohlenhydrat und gegebenenfalls Fett enthaltenden Nährstoffzusammensetzung umfaßt, **dadurch gekennzeichnet**, daß die Kohlenhydratkomponente der Nährstoffzusammensetzung ein thermogenetisches Gemisch von Stärke (St.), Rohrzucker (Su) und Glucose (Gl) umfaßt.

2. Oral einnehmbare Zusammensetzung nach Anspruch 1, **dadurch gekennzeichnet**, daß die Kohlenhydratkomponente der Nährstoffzusammensetzung ein Gemisch ist, das im wesentlichen aus Stärke, Rohrzucker und Glucose in einem Gewichtsverhältnis von 6,4 : 3 : 1 oder 10,6 : 5 : 1 besteht.

3. Oral einnehmbare Zusammensetzung nach Anspruch 1 oder 2, in welcher die Nährstoffzusammensetzung auf einer Gewichtsbasis 50 bis 60 % Kohlenhydrat, 15 bis 50 % Protein und 0 bis 25 % Fett enthält.

4. Oral einnehmbare Zusammensetzung nach Anspruch 1, 2 oder 3, worin der Adenosinrezeptorantagonist eine Verbindung der Formel

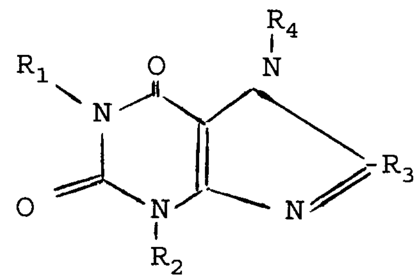

ist, worin $R_1$ und $R_2$ jeweils ein Wasserstoffatom oder eine $C_1$-$C_6$-Alkylgruppe ist, $R_4$ ein Wasserstoffatom oder eine Methylgruppe ist und $R_3$

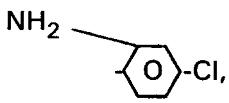, 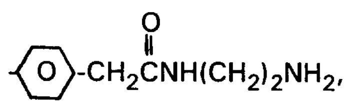, 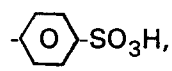, 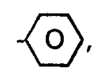, 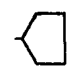

oder H ist, wobei, wenn $R_1$ und $R_2$ beides Methylgruppen sind, $R_3$ und $R_4$ nicht beide Wasserstoff bedeuten, und wenn $R_1$, $R_3$ und $R_4$ alle H sind, $R_2$ nicht Propyl ist.

**5.** Oral einnehmbare Zusammensetzung nach Anspruch 1, 2 oder 3, worin der Adenosinrezeptorantagonist
8-Cyclopentyltheophyllin (CPT),
8-Phenyltheophyllin (8-PT),
8-[4-(2-Aminoethyl)-amino]-carboxylmethyloxyphenyl]-1,3-dipropylxanthin (XAC),
8-(2-Amino-4-chlorphenyl)-1,3-dipropylxanthin (PACPX),
8-(p-Sulfophenyl)-1,3-dipropoylxanthin (DPSPX) oder
Theobromin (3,7-Dimethylxanthin) ist.

**6.** Oral einnehmbare Zusammensetzung nach Anspruch 1, 2 oder 3, die ein Gemisch von Stärke, Rohrzucker, Glucose und Kakao umfaßt.

## Revendications

**1.** Composition absorbable par voie orale à employer pour augmenter de la tolérance de l'homme ou des animaux à un milieu froid ou dans le traitement de l'hypothermie provoquée par l'exposition du corps humain ou du corps animal à un milieu froid, ladite composition comportant une quantité efficace du point de vue thermogène d'un inhibiteur des récepteurs adénosine, autre que la théophylline ou l'aminophylline seule, en mélange avec une composition nutritive contenant protéine, glucide et éventuellement matière grasse, caractérisée en ce que le composant glucidique de la composition nutritive comporte un mélange thermogène d'amidon (St.), de sucrose (Su) et de glucose (Gl).

**2.** Composition absorbable par voie orale selon la revendication 1, caractérisée en ce que le composant glucidique de la composition nutritive est un mélange essentiellement constitué par de l'amidon, du sucrose et du glucose dans une proportion en poids de 6:4:3:1 ou 10:6:5:1.

**3.** Composition absorbable par voie orale selon la revendication 1 ou 2, dans laquelle la composition nutritive contient en poids, 50 à 60% de glucide, 15 à 50% de protéine et 0 à 25% de matière grasse.

**4.** Composition absorbable par voie orale selon la revendication 1, 2 ou 3, dans laquelle l'inhibiteur des récepteurs adénosine est un composé de formule

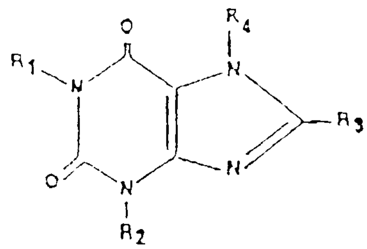

où $R_1$ et $R_2$ sont chacun un atome d'hydrogène ou un groupe alkyle en $C_1$-$C_6$,
$R_4$ est un atome d'hydrogène ou un groupe méthyle,
et $R_3$ est

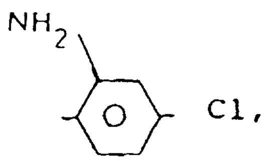

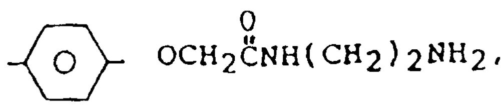

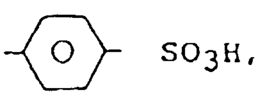

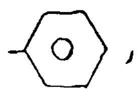

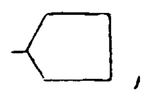

ou H ; à condition que lorsque $R_1$ et $R_2$ sont tous les deux un groupe méthyle, que $R_3$ et $R_4$ ne soient pas tous les deux un atome d'hydrogène et que lorsque $R_1$, $R_3$ et $R_4$ sont tous H, que $R_2$ ne soit pas un groupe propyle.

**5.** Composition absorbable par voie orale selon la revendication 1, 2 ou 3, dans laquelle l'inhibiteur des récepteurs adénosine est
8-cyclopentylthéophylline (CPT) ;
8-phénylthéophylline (8-PT) ;
8-(4-(2-aminoéthyl)amino)carboxylméthyloxyphényl)-1,3-dipropylxanthine (XAC) ;
8-(2-amino-4-chlorophényl)-1,3-dipropylxanthine (PACPX) ;
8-(p-sulfophényl)-1,3-dipropylxanthine (DPSPX) ;
théobromine (3,7-diméthylxanthine).

**6.** Composition absorbable par voie orale selon la revendication 1, 2 ou 3, qui comporte un mélange d'amidon, de sucrose, de glucose et de cacao.

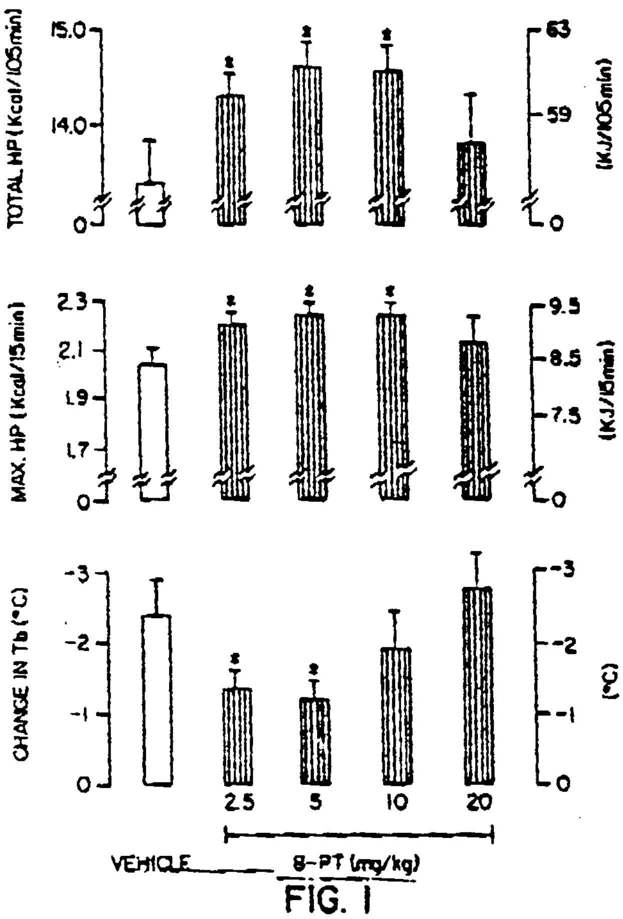
TOTAL HP (Kcal/105min)
15.0
14.0
0
63
59
0
(KJ/105min)
MAX. HP (Kcal/15min)
2.3
2.1
1.9
1.7
0
9.5
8.5
7.5
0
(KJ/15min)
CHANGE IN Tb (°C)
-3
-2
-1
0
(°C)
2.5
5
10
20
VEHICLE
8-PT (mg/kg)

FIG. 1

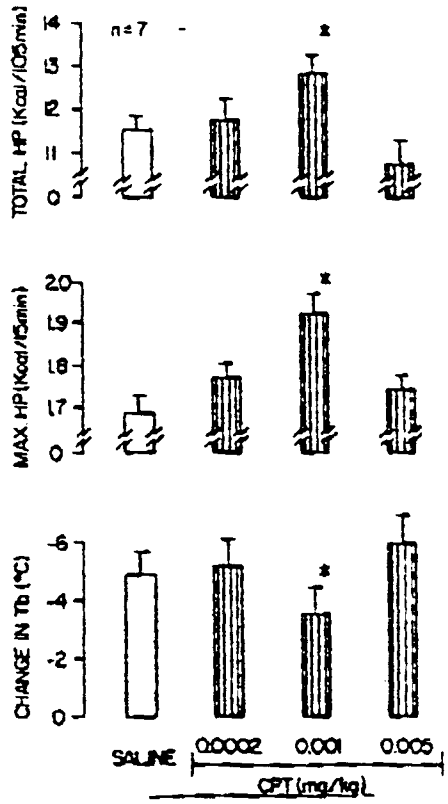
TOTAL HP (Kcal/105min)
n=7
14
13
12
11
0
MAX. HP (Kcal/15min)
2.0
1.9
1.8
1.7
0
CHANGE IN Tb (°C)
-6
-4
-2
0
SALINE
0.0002
0.001
0.005
CPT (mg/kg)

FIG. 2

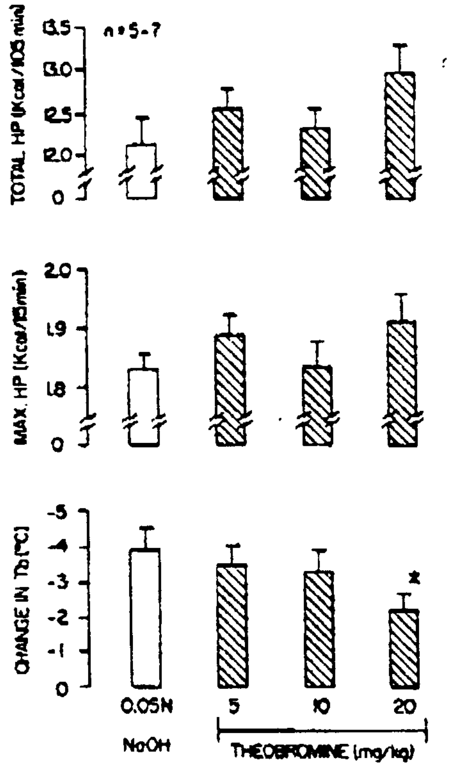
TOTAL HP (Kcal/105 min)
n=5-7
13.5
13.0
12.5
12.0
0
MAX. HP (Kcal/15 min)
2.0
1.9
1.8
0
CHANGE IN Tb (°C)
-5
-4
-3
-2
-1
0
0.05N
NaOH
5
10
20
THEOBROMINE (mg/kg)

FIG. 4

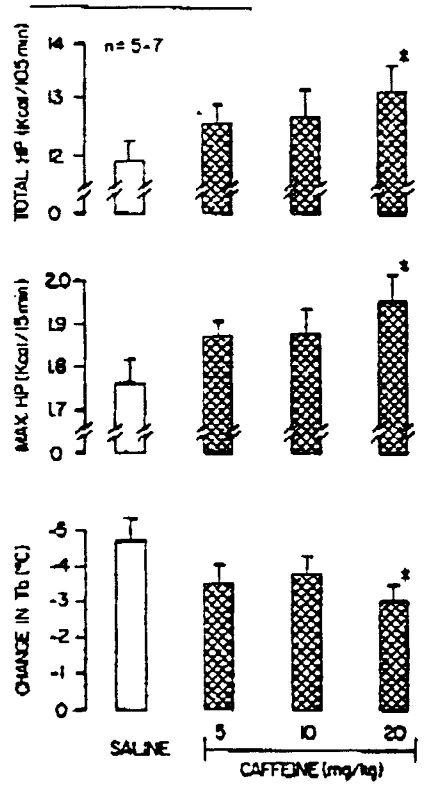
TOTAL HP (Kcal/105 min)
n= 5-7
14
13
12
0
MAX. HP (Kcal/15 min)
2.0
1.9
1.8
1.7
0
CHANGE IN Tb (°C)
-5
-4
-3
-2
-1
0
SALINE
5
10
20
CAFFEINE (mg/kg)

FIG. 3

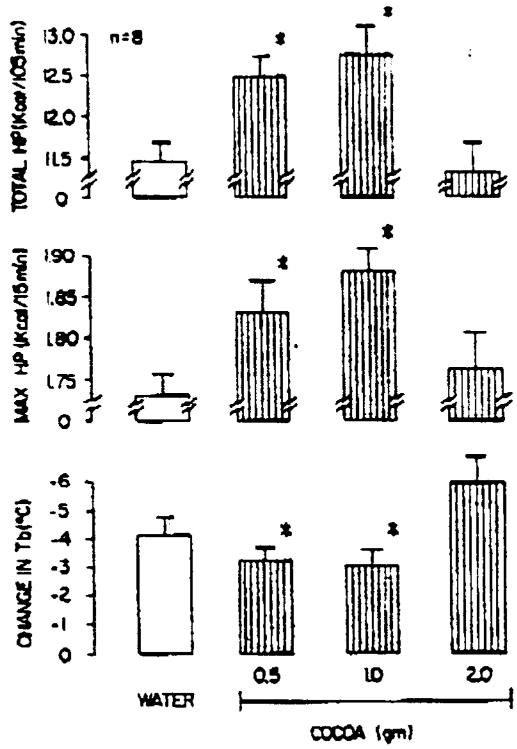
TOTAL HP (Kcal/105min)
MAX HP (Kcal/15min)
CHANGE IN Tb(°C)
n=8
WATER
0.5
1.0
2.0
COCOA (gm)

FIG. 5

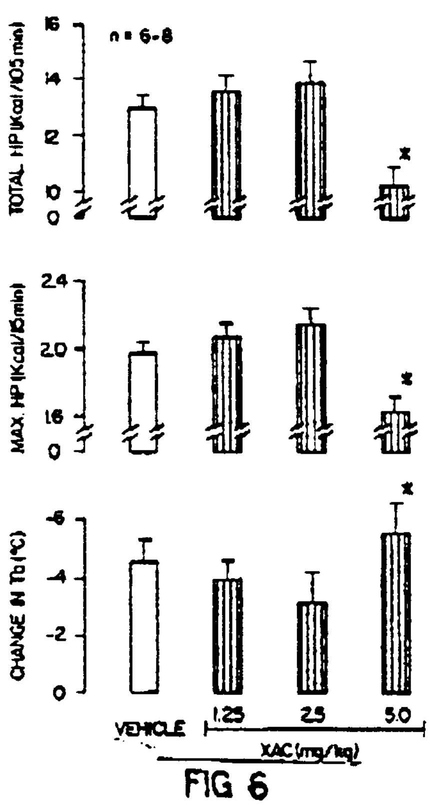
TOTAL HP (Kcal/105 min)
MAX. HP (Kcal/15min)
CHANGE IN Tb (°C)
n = 6-8
VEHICLE
1.25
2.5
5.0
XAC (mg/kg)

FIG 6

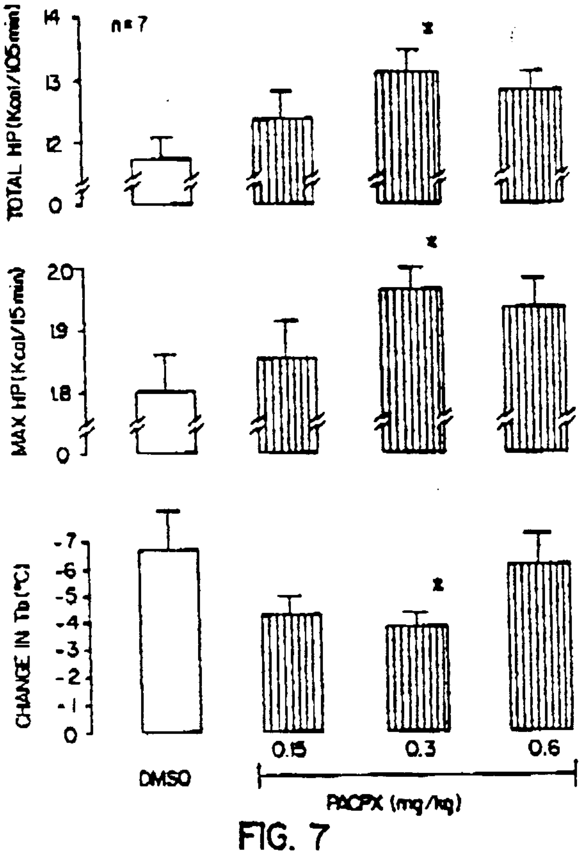
n=7
TOTAL HP (Kcal/105 min)
14
13
12
0
MAX HP (Kcal/15 min)
2.0
1.9
1.8
0
CHANGE IN Tb (°C)
-7
-6
-5
-4
-3
-2
-1
0
0.15
0.3
0.6
DMSO
PACPX (mg/kg)

FIG. 7

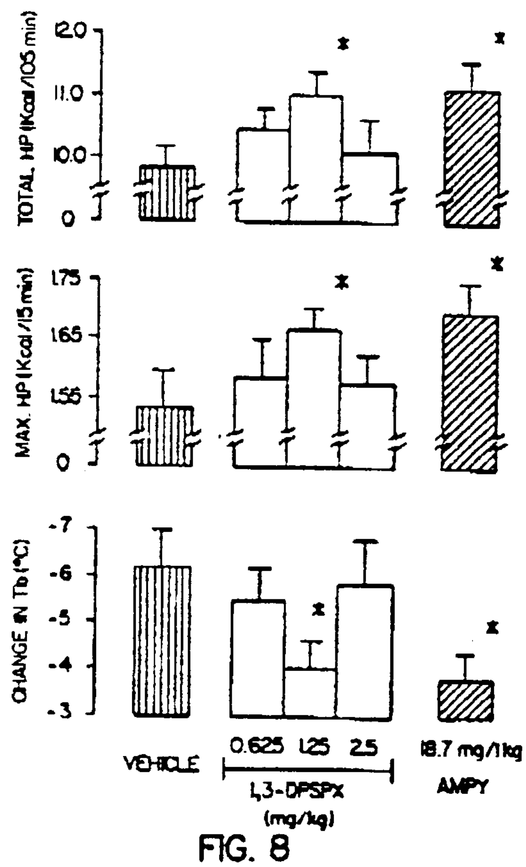
TOTAL HP (Kcal/105 min)
12.0
11.0
10.0
0
MAX. HP (Kcal/15 min)
1.75
1.65
1.56
0
CHANGE IN Tb (°C)
-7
-6
-5
-4
-3
0.625
1.25
2.5
18.7 mg/1kg
VEHICLE
1,3-DPSPX
(mg/kg)
AMPY

FIG. 8

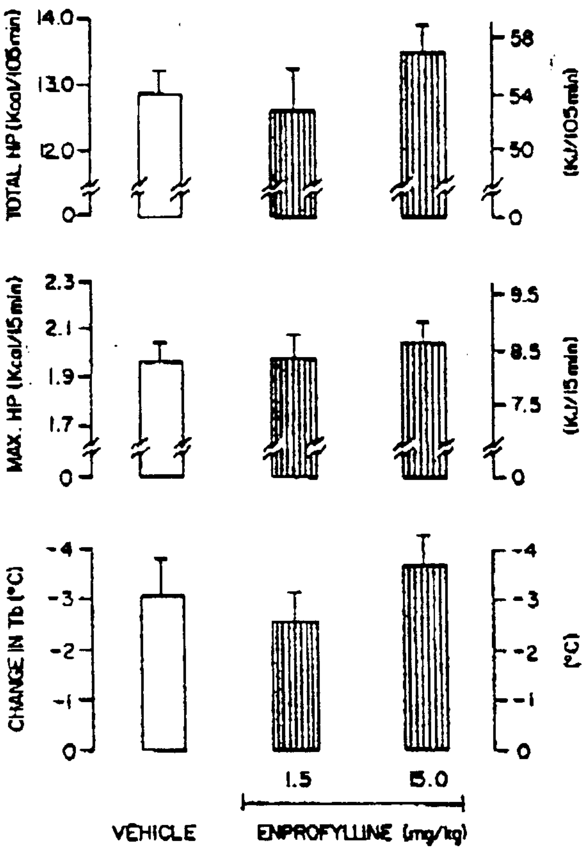
TOTAL HP (Kcal/105min)
(KJ/105min)
MAX. HP (Kcal/15min)
(KJ/15min)
CHANGE IN Tb (°C)
(°C)
1.5
15.0
VEHICLE
ENPROFYLLINE (mg/kg)

FIG. 9

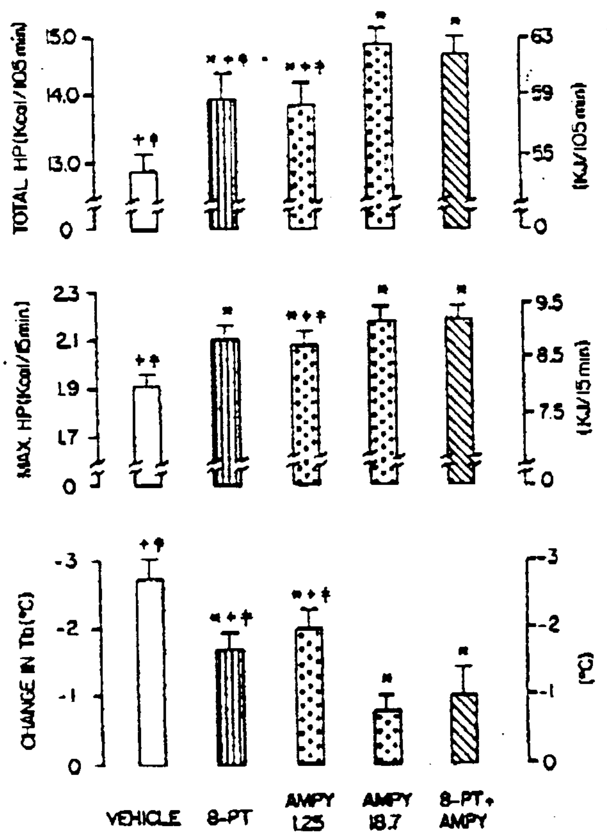
TOTAL HP (Kcal/105min)
(KJ/105min)
MAX. HP (Kcal/15min)
(KJ/15min)
CHANGE IN Tb (°C)
(°C)
VEHICLE
8-PT
AMPY 1.25
AMPY 18.7
8-PT+ AMPY

FIG 10

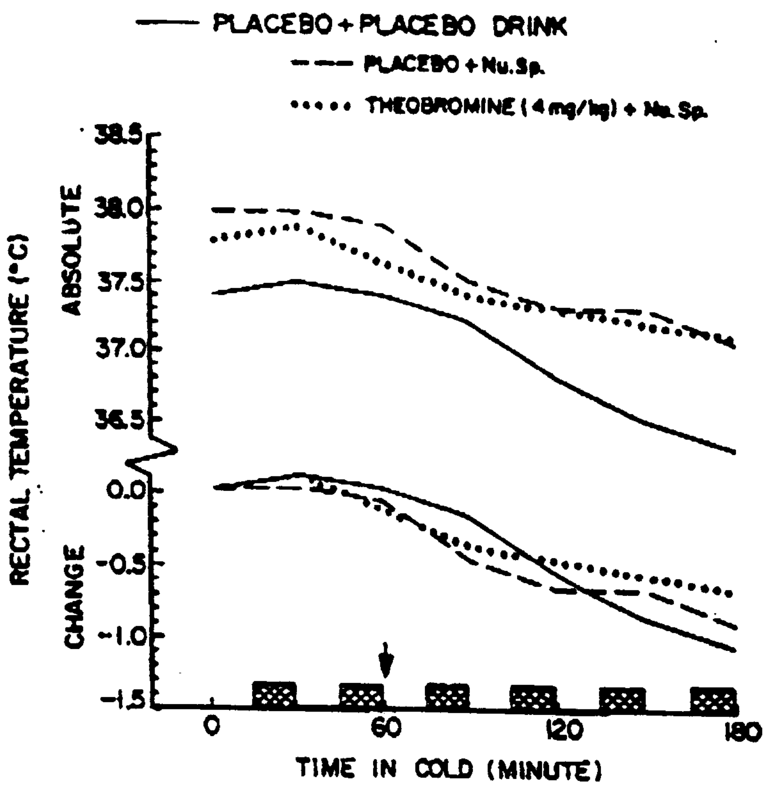
PLACEBO + PLACEBO DRINK
PLACEBO + Nu.Sp.
THEOBROMINE (4 mg/kg) + Nu.Sp.
RECTAL TEMPERATURE (°C)
ABSOLUTE
CHANGE
38.5
38.0
37.5
37.0
36.5
0.0
-0.5
-1.0
-1.5
0
60
120
180
TIME IN COLD (MINUTE)

FIG. 12

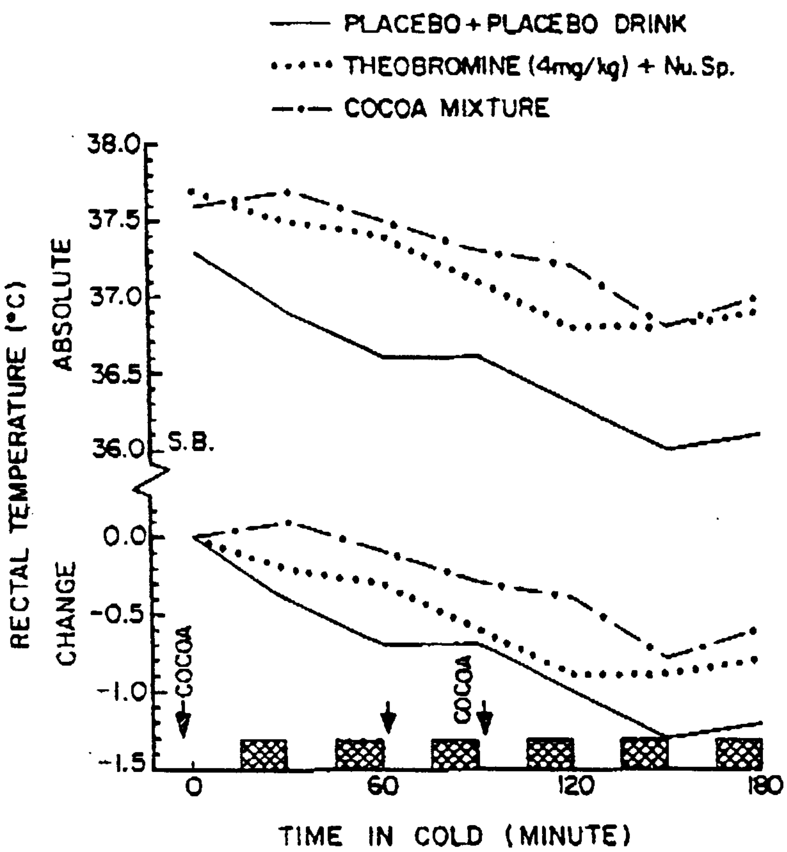
PLACEBO + PLACEBO DRINK
THEOBROMINE (4mg/kg) + Nu.Sp.
COCOA MIXTURE
RECTAL TEMPERATURE (°C)
ABSOLUTE
CHANGE
38.0
37.5
37.0
36.5
36.0
S.B.
0.0
-0.5
-1.0
-1.5
COCOA
COCOA
0
60
120
180
TIME IN COLD (MINUTE)

FIG. 13

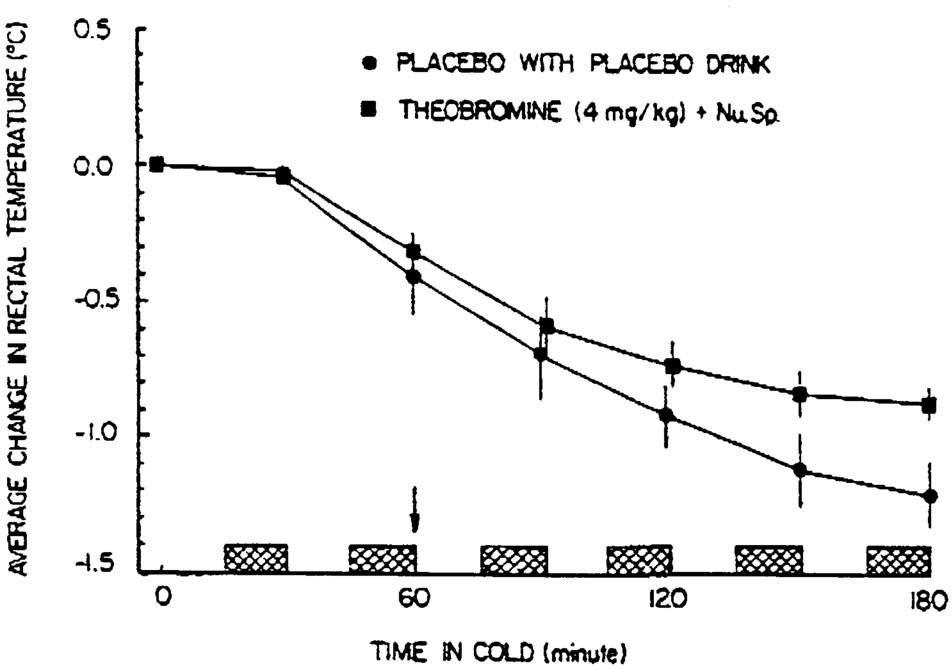
AVERAGE CHANGE IN RECTAL TEMPERATURE (°C)
0.5
0.0
-0.5
-1.0
-1.5
PLACEBO WITH PLACEBO DRINK
THEOBROMINE (4 mg/kg) + Nu.Sp.
0
60
120
180
TIME IN COLD (minute)

FIG. 14

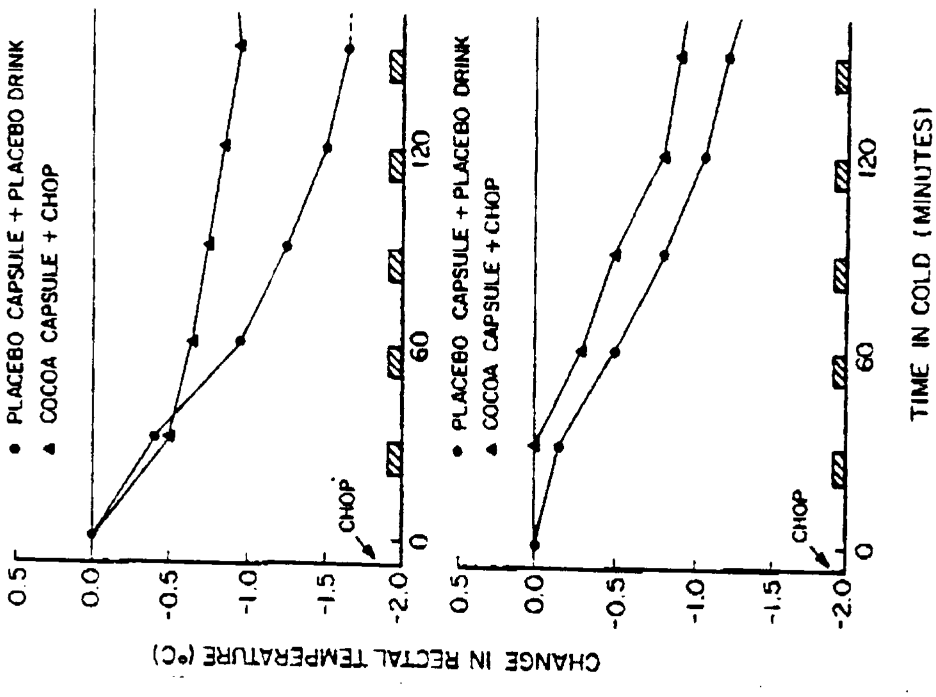
PLACEBO CAPSULE + PLACEBO DRINK
COCOA CAPSULE + CHOP
PLACEBO CAPSULE + PLACEBO DRINK
COCOA CAPSULE + CHOP
CHOP
CHOP
CHANGE IN RECTAL TEMPERATURE (°C)
0.5
0.0
-0.5
-1.0
-1.5
-2.0
0
60
120
TIME IN COLD (MINUTES)

FIG. 16

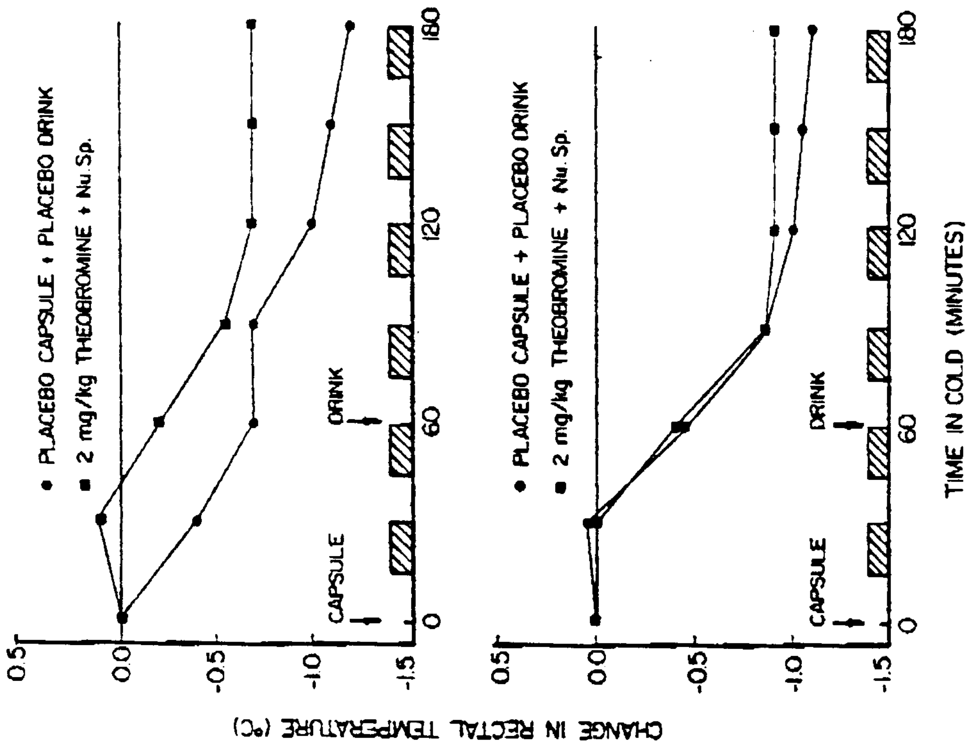
PLACEBO CAPSULE + PLACEBO DRINK
2 mg/kg THEOBROMINE + Nu.Sp.
PLACEBO CAPSULE + PLACEBO DRINK
2 mg/kg THEOBROMINE + Nu.Sp.
CAPSULE
DRINK
CAPSULE
DRINK
CHANGE IN RECTAL TEMPERATURE (°C)
0.5
0.0
-0.5
-1.0
-1.5
0
60
120
180
TIME IN COLD (MINUTES)

FIG. 15